(19) Europäisches Patentamt
European Patent Office
Office européen des brevets

(11) **EP 2 057 990 A1**

(12) **EUROPEAN PATENT APPLICATION**
published in accordance with Art. 153(4) EPC

(43) Date of publication:
**13.05.2009 Bulletin 2009/20**

(51) Int Cl.:
**A61K 33/00** (2006.01)    **A23L 1/30** (2006.01)
**A23L 2/38** (2006.01)    **A61K 9/08** (2006.01)
**A61K 9/72** (2006.01)    **A61P 3/06** (2006.01)
**A61P 9/10** (2006.01)

(21) Application number: **07806899.6**

(22) Date of filing: **31.08.2007**

(86) International application number:
**PCT/JP2007/067458**

(87) International publication number:
**WO 2008/026785 (06.03.2008 Gazette 2008/10)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **31.08.2006 JP 2006235922**

(71) Applicants:
• **Ohta, Shigeo**
**Nakahara-ku**
**Kawasaki-shi, Kanagawa 211-0011 (JP)**

• **Murota, Wataru**
**Tsubata-machi**
**Kahoku-gun, Ishikawa 929-0324 (JP)**

(72) Inventor: **OHSAWA, Ikuroh**
**Shinjuku-ku**
**Tokyo 161-0032 (JP)**

(74) Representative: **Denison, Christopher Marcus et al**
**Mewburn Ellis LLP**
**33 Gutter Lane**
**London**
**EC2V 8AS (GB)**

(54) **LIPID METABOLISM IMPROVING AGENT CONTAINING HYDROGEN MOLECULE**

(57)    According to the present invention, a lipid metabolism improving agent containing hydrogen molecules that is in a liquid or gas state is provided.

The lipid metabolism improving agent of the present invention comprises a liquid or gas containing at least hydrogen molecules. It may comprise a medium containing oxygen molecules. Further, such medium may comprise water, an aqueous solution, or a gas. The lipid metabolism improving agent has effects of suppressing body fat accumulation, arteriosclerosis, and the like.

EP 2 057 990 A1

# EP 2 057 990 A1

**Description**

Technical Field

The present invention relates to a lipid metabolism improving agent for suppression of body fat ccumulation, arteriosclerosis, and the like, which contains, as active ingredients, hydrogen molecules.

Background Art

Fat is an important nutrient, and it is particularly important as an energy source. However, excessive fat ingestion promotes obesity and causes lifestyle diseases such as arteriosclerosis, which is problematic. In food-satiated developed countries, lifestyle diseases due to excessive fat ingestion are seriously problematic.

In order to prevent lifestyle diseases such as arteriosclerosis, it is necessary to improve lipid metabolism by, for example, suppressing fat accumulation and decreasing serum cholesterol concentrations. Foods and agents exhibiting a variety of lipid metabolism improving effects have been reported.

For instance, diglyceride is known to have many physiological activities that result in serum triglyceride decreasing effects, weight increase suppressing effects, and the like (see Patent Document 1). In addition, it has been reported that catechins in tea have effects of promoting the burning of accumulated body fat and of decreasing neutral fat and total cholesterol (see Patent Document 2).

Meanwhile, it has been suggested that a hydrogen gas is effective for health promotion (see Patent Document 3). Specifically, the use of a hydrogen-containing agent for inflammation treatment has been reported (see Patent Document 4). However, there have been no reports regarding the association between hydrogen molecules and lipid metabolism.

Patent Document 1: JP Patent Publication (Kokai) No. 2002-322052 A
Patent Document 2: JP Patent Publication (Kokai) No. 2002-326932 A
Patent Document 3: JP Patent Publication (Kokai) No. 2005-87257 A
Patent Document 4: JP Patent Publication (Kohyo) No. 2000-517311 A

Disclosure of the Invention

It is an objective of the present invention to provide a lipid metabolism improving agent containing hydrogen molecules that is in a liquid or gas state. The lipid metabolism improving agent functions as a body fat accumulation suppressor or an arteriosclerosis suppressor.

The present inventors conducted intensive studies of various *in vivo* effects of hydrogen molecules. The present inventors found in the past that hydrogen molecules have effects of removing toxic *in vivo* reactive oxygen species and/or free radicals. They have already filed a patent application for an agent for removing toxic *in vivo* reactive oxygen species and/or free radicals that comprises hydrogen molecules (the PCT international patent application based on JP Patent Application Nos. 2005-2385725 and 2006-157827).

Further, the present inventors have examined *in vivo* effects of hydrogen molecules. Accordingly, they have found that *in vivo* ingestion of a liquid or gas containing hydrogen molecules results in prevention and treatment of lipid-metabolism-related abnormalities. Such effects include reduction in serum LDL cholesterol and accumulated fat and improvement of arteriosclerosis. In addition, they first found that hydrogen molecules improve lipid metabolism. The present inventors used, as a lipid metabolism improving agent, a composition containing hydrogen molecules that is in a liquid or gas state. This has led to the completion of the present invention.

Specifically, the present invention is described as follows.

[1] A lipid metabolism improving agent comprising a liquid containing at least hydrogen molecules.

[2] The lipid metabolism improving agent according to [1], wherein the liquid containing at least hydrogen molecules is an aqueous solution.

[3] The lipid metabolism improving agent according to [2], which contains hydrogen molecules at 0.1 ppm (0.05 mM) or more.

[4] The lipid metabolism improving agent according to [2] or [3], which further contains oxygen molecules.

[5] A lipid metabolism improving agent comprising a gas containing at least hydrogen molecules.

[6] The lipid metabolism improving agent according to [5], wherein the gas containing at least hydrogen molecules is a gas mixture of a hydrogen gas and an oxygen gas.

[7] The lipid metabolism improving agent according to [5], wherein the gas containing at least hydrogen molecules is a gas mixture of a hydrogen gas, an oxygen gas, and an inert gas.

[8] The lipid metabolism improving agent according to [5], wherein the gas containing at least hydrogen molecules is a gas mixture of a hydrogen gas and air.

[9] The lipid metabolism improving agent according to any one of [5] to [8], which contains a hydrogen gas at a concentration of 1% to 4% (v/v).

[10] The lipid metabolism improving agent according to any one of [5] to [8], which contains a hydrogen gas at a concentration of 1.0% to 4.5% (v/v).

[11] The lipid metabolism improving agent according to any one of [1] to [10], which suppresses body fat accumulation.

[12] The lipid metabolism improving agent according to any one of [1] to [10], which suppresses arteriosclerosis.

[13] The lipid metabolism improving agent according to any one of [1] to [10], which reduces low density lipoprotein (LDL) cholesterol in blood.

[14] A beverage suitable for lipid metabolism improvement, which comprises a liquid containing at least hydrogen molecules.

[15] The beverage suitable for lipid metabolism improvement according to [14], wherein the liquid containing at least hydrogen molecules is an aqueous solution.

[16] The beverage suitable for lipid metabolism improvement according to [15], which contains hydrogen molecules in an oversaturated state.

[17] The beverage suitable for lipid metabolism improvement according to [14] or [15], which further contains oxygen molecules.

[18] The beverage according to any one of [14] to [17], which is labeled for use for improving lipid metabolism.

[19] The beverage according to [18], which is labeled for use for suppressing body fat accumulation.

[20] The beverage according to [18], which is labeled for use for suppressing arteriosclerosis.

[21] The beverage according to [18], which is labeled for use for reducing low density lipoprotein (LDL) cholesterol in blood.

[22] The beverage according to any one of [14] to [21], which corresponds to a health food, a functional food, a nutritional supplement food, a supplement, or a specified health food.

[23] A container containing the lipid metabolism improving agent according to any one of [5] to [10].

[24] The container according to [23], which is a hydrogen gas cylinder.

[25] An apparatus for supplying the lipid metabolism improving agent comprising a gas containing at least hydrogen molecules according to any one of [5] to [10] to a test subject that needs to improve lipid metabolism, which comprises a container containing the lipid metabolism improving agent, a gas inhalation means, and a supply pipe for supplying a gas in the container to the inhalation means.

[26] The apparatus for supplying the lipid metabolism improving agent to a test subject that needs to improve lipid metabolism according to [25], which comprises a container containing at least one type of gas selected from the group consisting of an oxygen gas, an inert gas, and air, provided that the lipid metabolism improving agent comprising a gas containing at least hydrogen molecules and at least one type of gas selected from the group consisting of an oxygen gas, an inert gas, and air are supplied separately or in a mixed state to the gas inhalation means.

[27] The apparatus for supplying the lipid metabolism improving agent to a test subject that needs to improve lipid metabolism according to [25] or [26], which contains a hydrogen gas at 1% to 4% in the container containing the lipid metabolism improving agent comprising a gas containing at least hydrogen molecules.

[28] The apparatus for supplying the lipid metabolism improving agent to a test subject that needs to improve lipid metabolism according to any one of [25] to [27], wherein the container containing the lipid metabolism improving agent comprising a gas containing at least hydrogen molecules is a gas cylinder.

[29] The apparatus for supplying the lipid metabolism improving agent comprising a gas containing at least hydrogen molecules to a test subject that needs to improve lipid metabolism according to any one of [25] to [28], wherein the gas inhalation means is a gas inhalation mask.

[30] The apparatus for supplying the lipid metabolism improving agent to a test subject that needs lipid metabolism improvement according to any one of [25] to [28], wherein the gas inhalation means is a sealed chamber, provided that the lipid metabolism improving agent comprising a gas containing at least hydrogen molecules is supplied to the sealed chamber such that the lipid metabolism improving agent is supplied to the test subject in the sealed chamber.

This description includes part or all of the contents as disclosed in the description and/or drawings of Japanese Patent Application No. 2006-235922, which is a priority document of the present application.

Brief Description of the Drawings

Fig. 1 shows an image of a water supply bottle for administration of hydrogen water to mice.

Fig. 2 shows an apparatus for administration of hydrogen gas to mice.

Fig. 3 shows stained liver fat images of a mouse to which hydrogen water was administered and mice to which the same was not administered.

Fig. 4 shows liver fat accumulation in mice to which hydrogen water was administered and that in mice to which the same was not administered.

Fig. 5 shows Oil-Red-O-stained artery images of an apoE-KO mouse to which hydrogen water was administered and an apoE-KO mouse to which the same was not administered.

Fig. 6 shows artery thickening in apoE-KO mice to which hydrogen water was administered and that in apoE-KO mice to which the same was not administered.

Fig. 7 shows weight changes in apoE-KO mice to which hydrogen water was administered and apoE-KO mice to which the same was not administered.

Fig. 8 shows the muscle force of apoE-KO mice to which hydrogen water was administered and that of apoE-KO mice to which the same was not administered.

Fig. 9 shows images representing results of body fat analysis by CT scan for an apoE-KO mouse to which hydrogen water was administered and an apoE-KO mouse to which the same was not administered.

Fig. 10 shows a graph representing muscle and fat weights calculated based on CT images of apoE-KO mice to which hydrogen water was administered and apoE-KO mice to which the same was not administered.

Fig. 11 shows a graph representing fat percentages calculated based on CT images of apoE-KO mice to which hydrogen water was administered and apoE-KO mice to which the same was not administered.

Fig. 12 shows the individual organ weights of apoE-KO mice to which hydrogen water was administered and apoE-KO mice to which the same was not administered (12 weeks after hydrogen gas administration).

Fig. 13A shows images of liver fat accumulation of an apoE-KO mouse to which hydrogen water was administered and an apoE-KO mouse to which the same was not administered (12 weeks after hydrogen gas administration).

Fig. 13B shows the accumulated liver fat amounts of an apoE-KO mouse to which hydrogen water was administered and an apoE-KO mouse to which the same was not administered (12 weeks after hydrogen gas administration).

Fig. 14 shows a graph representing effects of suppressing mouse serum LDL cholesterol and arteriosclerosis index, which were induced by hydrogen water ingestion.

Fig. 15 shows an apparatus for supplying the lipid metabolism improving agent containing hydrogen gas of the present invention.

Explanation of Reference Numerals

1: Gas inhalation means
2: Container containing a lipid metabolism improving agent comprising a gas containing hydrogen molecules
3: Container containing gas
4: Pipe

Best Mode for Carrying Out the Invention

The lipid metabolism improving agent of the present invention comprises a liquid or gas containing at least hydrogen molecules.

According to the present invention, the term "lipid metabolism improvement" refers to improvement or prevention of various forms of health damage resulting from fat accumulation in the body induced by environmental factors such as diet, exercise, and aging and genetic factors such as metabolic abnormalities.

Lipid metabolism improvement includes suppression of body fat accumulation, anti-obesity, and suppression of weight increase. Herein, suppression of accumulation includes prevention of fat accumulation and reduction of accumulated fat. The term "suppression of body fat accumulation" includes suppression of visceral fat accumulation, suppression of subcutaneous fat accumulation, and suppression of fatty liver formation. In addition, the lipid metabolism improving agent of the present invention can promote the burning of fat accumulated in the body and of ingested dietary fat.

Further, the term "lipid metabolism improvement" includes suppression of serum cholesterol concentration, serum triglyceride concentration increase, hepatocellular cholesterol synthesis, and ACAT activity. Serum cholesterol is roughly classified as LDL (bad) cholesterol and HDL (good) cholesterol. Based on many epidemiological surveys, it is known that an increase in serum LDL cholesterol concentration promotes the advancement of ischemic heart diseases and the like. Meanwhile, HDL cholesterol removes cholesterol accumulated on blood vessel walls and thus it is antagonistic to LDL cholesterol, which transports cholesterol into blood vessels. Thus, HDL cholesterol is known to have effects of preventing ischemic heart diseases and the like. The lipid metabolism improving agent of the present invention has substantially no effects of decreasing the serum HDL cholesterol concentration. However, it can selectively decrease the serum LDL cholesterol concentration. That is, the lipid metabolism improving agent of the present invention has effects of decreasing the total serum cholesterol concentration and also has effects of improving the abundance ratio of LDL (bad) cholesterol to HDL (good) cholesterol.

Furthermore, the lipid metabolism improving agent of the present invention has effects of suppressing arteriosclerosis and of reducing the arteriosclerosis index. Thus, it can be used for treating, preventing, or improving arteriosclerosis. Herein, the term "arteriosclerosis index" refers to an index represented by the following equation in which "LDL-C" denotes the low density lipoprotein cholesterol concentration and "HDL-C" denotes the high density lipoprotein cholesterol concentration.

$$\text{Arteriosclerosis index} = [(\text{total cholesterol}) - (\text{HDL-C})] \div (\text{HDL-C})$$

Further, since the lipid metabolism improving agent of the present invention can improve lipid metabolism as described above, it can also be used for treating, preventing, or improving diseases such as hypertension, hyperlipidemia, arteriosclerosis, myocardial infarction, brain blood vessel disorders, and metabolic syndromes.

Therefore, the lipid metabolism improving agent of the present invention also functions as a body fat accumulation suppressor, an arteriosclerosis suppressor, a blood LDL cholesterol suppressor, or the like.

In addition, at the same time, the lipid metabolism improving agent can be used as an agent for preventing or treating abnormalities such as diseases related to lipid metabolism abnormality. Examples of abnormalities such as diseases related to lipid metabolism abnormality include obesity, hyperlipidemia, kidney diseases, diabetes mellitus, thyroid dysfunction, and metabolic syndrome.

A liquid containing at least hydrogen molecules is characterized in that it comprises an aqueous solution. As a medium constituting the aqueous solution, pure water, ion exchange water, distilled water, physiological saline, and the like can be used. Further, a liquid containing hydrogen molecules obtained using, as the medium, pure water, ion exchange water, or distilled water may be added to a common aqueous beverage for drinking. Examples of such beverage include: mineral water; fruit juice/fruit beverages; coffee beverages; tea beverages such as oolong tea beverages, green tea beverages, black tea beverages, and barley tea beverages; vegetable beverages; and sport beverages. In addition, hydrogen molecules may be directly dissolved in mineral water, juice, coffee, tea, or the like. The beverage may contain the following additives which can be used alone or in combination: antioxidants, fragrances, a variety of esters, organic acids, organic acid salts, inorganic acids, inorganic acid salts, inorganic salts, pigments, emulsifiers, preservatives, seasonings, sweeteners, acidulants, fruit extracts, vegetable extracts, nectar extracts, pH adjusters, quality stabilizers, and the like. Herein, the term "beverage" includes health food, specified food for health use, nutritive functional food, and the like in beverage form. Herein, the term "specified food for health use" indicates food that is labeled for ingestion for specified health purposes for diet that can be expected to be achieved via ingestion of such food. These beverages may be labeled for use for improvement of lipid metabolism, suppression of body fat accumulation, promotion of fat burning, reduction of blood cholesterol, suppression of arteriosclerosis, and the like.

Hydrogen molecules can be dissolved in water or an aqueous solution for a certain time period even in an oversaturated state. Such water or aqueous solution containing oversaturated hydrogen molecules can be produced by dissolving a hydrogen gas in water or an aqueous solution under pressure and then depressurizing the resultant. For instance, an aqueous solution may be placed in a hydrogen gas at a pressure of 0.4 MPa or more for several hours, and preferably for 1 to 3 hours. Also, the lipid metabolism improving agent in an aqueous solution form can be used for drinking or intravenous injection when mixed with physiological saline. In such case, administration can be carried out with the use of catheters or by injection. After infusion, substantially all hydrogen ingested *in vivo* is absorbed in the body and transferred to the whole body via blood circulation. Then, hydrogen exhibits its effects and then is emitted by expiration.

Under conditions of a hydrogen pressure of 1 atmosphere and room temperature, approximately 17.5 mL of hydrogen can be dissolved in 1 L of water (approximately 1.6 ppm or approximately 0.8 mM). The amount of hydrogen molecules contained in 1L of the lipid metabolism improving agent in an aqueous solution form of the present invention is 1 mL or more, preferably 2 mL or more, further preferably 5 mL or more, 10 mL or more, or 15 mL or more, and particularly preferably 17.5 mL or more. In addition, the lipid metabolism improving agent in an aqueous solution form of the present invention contains hydrogen molecules at 0.1 ppm or more, preferably 0.2 ppm or more, and further preferably 0.5 ppm or more, 1 ppm or more, or 1.5 ppm or more. Moreover, the lipid metabolism improving agent in an aqueous solution form of the present invention contains hydrogen at 0.05 mM or more, preferably 0.1 mM or more, further preferably 0.2 mM or more, 0.4 mM or more, or 0.6 mM or more, and particularly preferably 0.8 mM or more.

Furthermore, the lipid metabolism improving agent in an aqueous solution form of the present invention may contain oxygen molecules. In such case, hydrogen molecules and oxygen molecules coexist in the aqueous solution. However, even in a case in which hydrogen molecules and oxygen molecules are mixed therein, they do not immediately react with each other, and thus both molecules can stably coexist. Note that, in a case in which a gas contains a large amount of oxygen molecules, it is preferable to predetermine the hydrogen content at less than 4.7% (v/v) with respect to the entire gas in line with safety requirements. Under use environments in which there are no safety problems, the hydrogen content is preferably as great as possible in terms of concentration. The lipid metabolism improving agent containing oxygen can be used for drinking or injection when it is mixed with physiological saline or the like. However, in the case of administration by injection, lack of oxygen is not locally induced *in vivo* compared with the case of an agent containing no oxygen. Thus, living tissues are less likely to be damaged.

The beverage of the present invention is preferably stored in a container made of a hydrogen-impermeable material, preferably such as aluminium. Examples of such container include aluminium pouches and aluminium cans. In addition, the lower the temperature, the greater the content of hydrogen dissolved. Thus, it is preferable to store the container at low temperatures.

The lipid metabolism improving agent containing at least hydrogen molecules of the present invention may be in a gas form. In such case, the hydrogen concentration is 1% to 4.7% (v/v), preferably 1% to 4.5% (v/v), more preferably 1% to 4% (v/v), further preferably 1.5% to 2.5% (v/v), and even further preferably approximately 2% (v/v). The hydrogen gas content is preferably approximately less than 4.7% (v/v) in line with safety requirements. However, under safe conditions in which sealing is achieved such that static electricity is not generated, the hydrogen gas content can be further increased. The lipid metabolism improving agent containing at least hydrogen molecules of the present invention may further contain an oxygen gas and/or a different inert gas. In a case in which an oxygen gas is contained, the agent comprises a gas mixture of a hydrogen gas and an oxygen gas. The oxygen gas is consumed for breathing. The inert gas that can be used is a nitrogen gas, a helium gas, an argon gas, or the like. However, a nitrogen gas, which is inexpensive, is preferable. The inert gas content can be arbitrarily determined by a person skilled in the art, unless it is excessive. In view of the oxygen gas concentration for breathing, the inert gas content is preferably 80% (v/v) or less. Further, the lipid metabolism improving agent containing at least hydrogen molecules of the present invention may be a gas mixture of a hydrogen gas and air. Such gas mixture can be readily produced by adequately mixing a hydrogen gas with air.

The lipid metabolism improving agent containing at least hydrogen molecules that is in a gas state of the present invention is placed in, for example, a pressure-proof container such as a gas cylinder. The present invention also encompasses a container containing the lipid metabolism improving agent containing at least hydrogen molecules that is in a gas state.

It is possible to allow a test subject to inhale the lipid metabolism improving agent containing at least hydrogen molecules that is in a gas state of the present invention. For inhalation, an inhalation means can be used. The inhalation route may be from the container containing the lipid metabolism improving agent through a pipe to an inhalation means. An inhalation means is not limited. However, for instance, an inhalation mask is used. Preferably, the mask can cover both the mouth and the nose of a test subject. Further, an example of an inhalation means is a small sealed chamber. Such small chamber has a size that can accommodate a test subject therein. In a condition in which a test subject is located inside a small chamber, the lipid metabolism improving agent containing at least hydrogen molecules that is in a gas state of the present invention is supplied to the small chamber. Thus, a test subject is allowed to inhale the agent. An example of such small chamber is a sealed bed. The test subject, while lying on the bed, can inhale the lipid metabolism improving agent containing at least hydrogen molecules that is in a gas state of the present invention. Such test subject needs to improve lipid metabolism and has lipid metabolism-related abnormalities, disorders, or the like.

In a case in which the lipid metabolism improving agent is in a liquid state, administration can be carried out by oral administration or intravenous injection. In addition, in a case in which the lipid metabolism improving agent is in a gas state, administration may be carried out by inhalation. Further, in both cases in which the lipid metabolism improving agent is in a liquid state and in which it is in a gas state, administration can be carried out by absorption through skin.

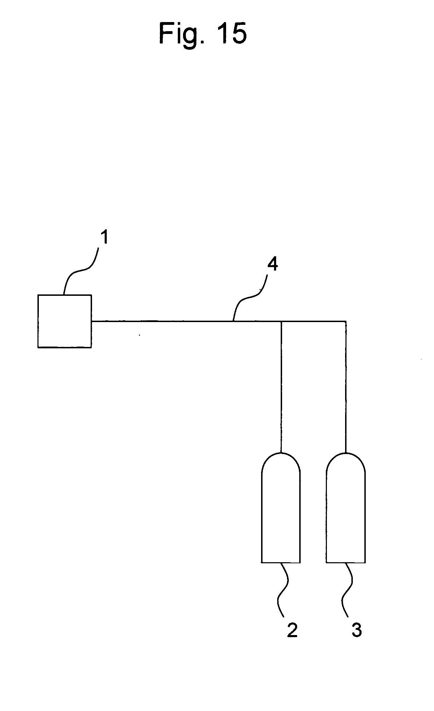

Further, the present invention encompasses an apparatus for supplying the lipid metabolism improving agent comprising a gas containing at least hydrogen molecules to a test subject that needs to improve lipid metabolism, such apparatus comprising a container containing the lipid metabolism improving agent, a gas inhalation means, and a supply pipe for supplying a gas in the container to the inhalation means. Such container is, for example, a hydrogen gas cylinder. In addition, as described above, examples of a gas inhalation means include an inhalation mask and a small sealed chamber. The apparatus may further comprise a container containing at least one type of gas selected from the group consisting of an oxygen gas, an inert gas, and air. In such case, the lipid metabolism improving agent comprising a gas containing at least hydrogen molecules and at least one type of gas selected from the group consisting of an oxygen gas, an inert gas, and air may be supplied separately or in a mixed state to the gas inhalation means. Fig 15 shows a schematic view of the apparatus of the present invention. In the figure, the apparatus comprises a gas inhalation means 1, a container 2 containing a lipid metabolism improving agent comprising a gas containing hydrogen molecules, a container 3 containing at least one type of gas selected from the group consisting of an oxygen gas, an inert gas, and air, and a pipe 4. The gas is supplied through the pipe to the gas inhalation means so as to be administered to a test subject.

The present invention is hereafter described in greater detail with reference to the following examples, although the technical scope of the present invention is not limited thereto. Various changes and modifications to the present invention can be made in an equal manner without departing from the spirit and scope thereof.

In the Examples, measurement of hydrogen concentration, preparation of hydrogen water, administration of hydrogen water to mice, and administration of hydrogen gas to mice were carried out as described below.

Measurement of hydrogen concentration

The hydrogen concentration in a solution was measured with a hydrogen electrode (ABLE Inc.). The hydrogen gas concentration was measured by gas chromatography (Teramecs Co., Kyoto).

Preparation of hydrogen water

For preparation of saturated hydrogen water, 1 liter of water was injected into a pressure-proof bottle with a volume of 5 liters (produced by Unicontrols Co., Ltd.) and then a hydrogen gas was introduced thereinto so as to result in a pressure of 0.4 megapascals. 2 hours later, hydrogenated water was recovered from the bottle with depressurization. Water (hydrogen water) containing saturated hydrogen at approximately 0.8 mM was obtained by the above process.

Administration of hydrogen water to mice

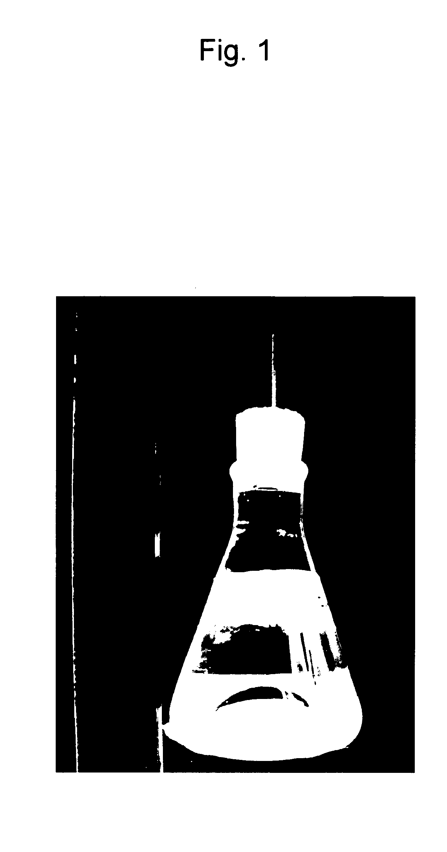

In an open system, dissolved hydrogen in hydrogen water disperses into the air and then disappears. Thus, in order to maintain hydrogen in a state such that it remains dissolved in water for a long time, a water ingestion valve accommodating 2 bearing balls was used (produced by Natsume Seisakusho Co., Ltd.) in a manner such that no water leaked and came into contact with air. A glass-made water supply bottle with a volume of approximately 150 mL was full-filled with hydrogen water and then a water ingestion valve was attached thereto (fig. 1). Each mouse was able to drink water from the water ingestion valve by pushing on the bearing balls. The water ingestion of a mouse in this method did not decrease to a level below that of an open system. In addition, with this method, the amount of dissolved hydrogen remained at a level corresponding to the half-saturation concentration or more even 24 hours later.

Administration of a hydrogen gas to mice

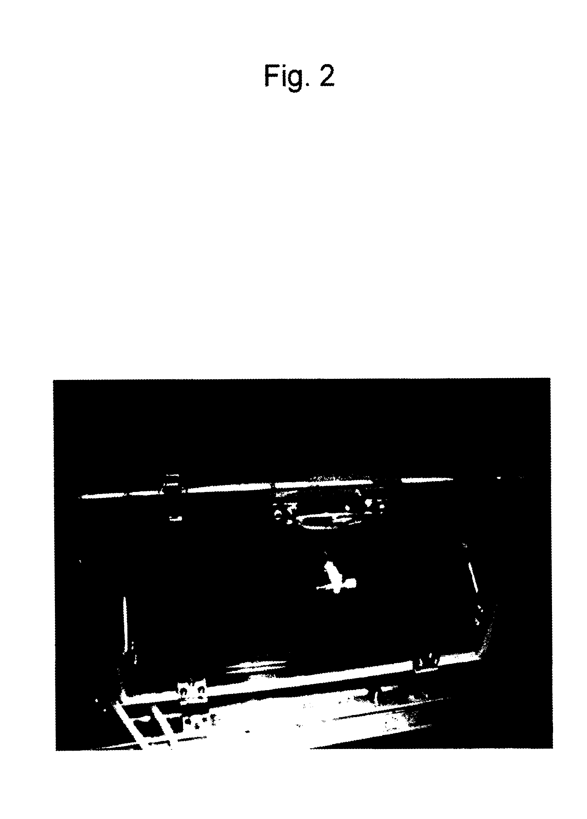

In order to allow each mouse to continuously inhale a hydrogen gas, pure hydrogen gas was mixed with the air delivered from a pump (produced by YASUNAGA AIR Pump Inc.) at a concentration of approximately 2% (1.5% to 2.5% volume/volume) and the resulting gas was sent into an airtight plastic container as shown in fig. 2. The size of this container was as follows: W: 550 mm; L: 400 mm; and H: 300 mm. The gas mixture containing the hydrogen gas was supplied thereinto at a rate of 2 to 5 liters per minute. The hydrogen gas concentration was measured according to the method described in "Measurement of hydrogen concentration" above. A cage in which mice were housed was placed in the container such that the hydrogen gas was stably administered to the mice.

Example 1: Effects of suppressing lipid accumulation by administering saturated hydrogen water to high-fat diet-fed mouse livers

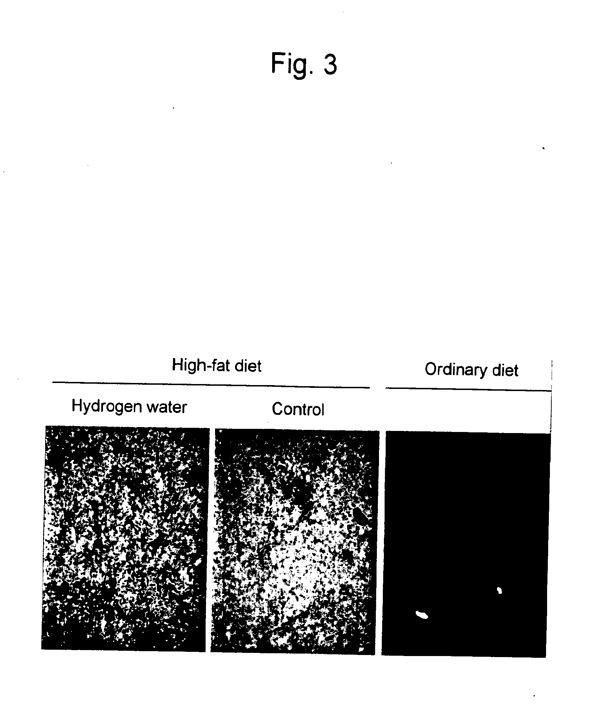

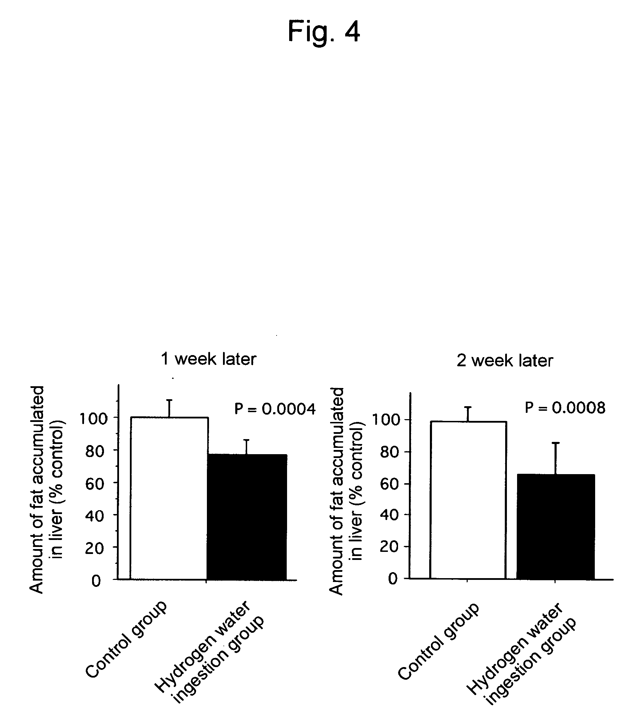

Normal mice (C57BL/6, male, 13 weeks old) were divided into 4 groups of 8 mice. Among them, 2 groups were fed ad libitum with hydrogen-free water (control groups), and the other 2 groups were fed ad libitum with hydrogen water (hydrogen water ingestion groups). The method for administrating hydrogen water was carried out according to the method described in "Administration of hydrogen water" above. Water used for control groups was obtained by stirring hydrogen water with a stirrer overnight so as to completely expel dissolved hydrogen. The water was replaced with fresh water once daily. For feed, the high-fat feed F2HFD1 (produced by Oriental Yeast Co., Ltd.) was supplied. The feed was replaced with fresh feed once daily. 1 week and 2 weeks later, each one group was subjected to ether anesthesia and livers were extracted therefrom, followed by fixation with 4% paraformaldehyde overnight and dehydration with sucrose. Thereafter, frozen sections were prepared and fat was specifically stained with Oil-Red O. Then, the amounts of lipid accumulated in livers were calculated. Fig. 3 shows stained images obtained 2 weeks later. Weaker staining was observed in the hydrogen water ingestion groups than in the control groups, indicating that fat accumulation had been suppressed. Substantially no staining was observed in normal livers of the groups to which an ordinary diet had been supplied. The graph in fig. 4 shows results of comparison of both groups, which were obtained by measuring stained areas with the use of free image processing software "NIH Image" based on digital images of the section surfaces. 1 week and 2 weeks later, it was shown that the amount of fat accumulated in the liver had more significantly decreased in the cases of the hydrogen water ingestion groups than in the cases of the control groups.

The above results indicate that effects of suppressing abnormal fat accumulation caused by excessive fat ingestion can be obtained by hydrogen water ingestion. In addition, such effects can be sufficiently exhibited in approximately 1 week.

Example 2: Administration of hydrogen water to arteriosclerosis animal models and effects of reducing arteriosclerosis lesions

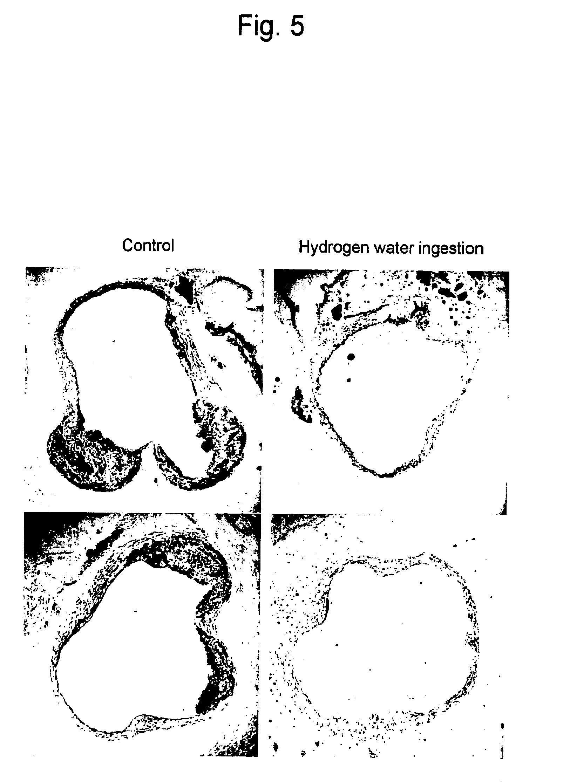

It is thought that arteriosclerosis is induced by incorporation of oxidized LDL into macrophages and accumulation of the resulting cells on arterial walls. Epidemiological surveys of humans have revealed that a risk factor of arteriosclerosis is the apolipoprotein E 4 gene (APOE4). In addition, arteriosclerosis is developed in apoE knockout mice (apoE-KO mice) even in a case in which the mice are fed with an ordinary diet for approximately 7 months. Therefore, the mice are widely used as animal models that develop arteriosclerosis (reference: FASEB J. 15, pp. 2623-2630, 2001; Circulation 110, pp. 1492-1498, 2004). Thus, hydrogen water was administered to apoE-KO mice and effects on arteriosclerosis lesions were examined. 5-week-old male apoE-KO mice were divided into a control group of 5 mice and a hydrogen water ingestion group of 7 mice. Hydrogen water was supplied to the latter group in the method described in Example 3. Water used for the control group was obtained by stirring hydrogen water with a stirrer overnight so as to completely expel dissolved hydrogen. The water was replaced with fresh water once daily. 28 weeks later, hearts were extracted under ether anesthesia. Serial sections (30 pieces) having a thickness of 10 micrometers were prepared from arteries within a 1-mm radius of the left ventricular valve. Every second section was selected and 15 pieces in total were stained with Oil-Red-O that specifically stains fat (fig. 5). Areas corresponding to artery-thickening regions stained with Oil-Red-O and cross-sectional areas of blood vessels were measured with the use of free image processing software "NIH Image" based on digital images of the section surfaces. The damaged volume was calculated by multiplying the measurement results by the thickness using the following equation.

$$\text{Damaged volume proportion (\%)} = \text{damaged volume/blood vessel volume} \times 100$$

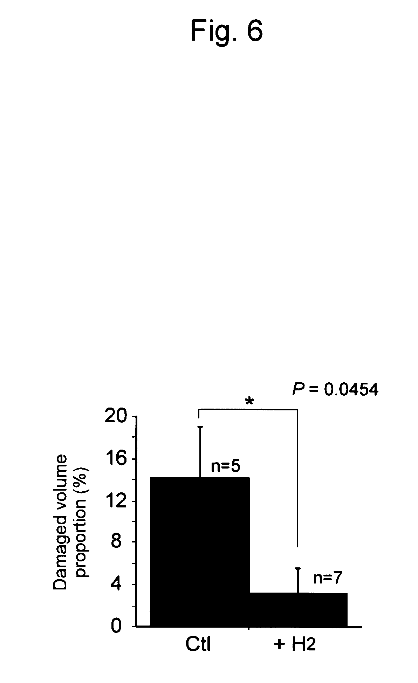

Artery thickening was observed in mice of the control group (fig. 5, left). However, artery thickening was not observed in 6 mice that were continuously allowed to drink hydrogen water (fig. 5, right). Among them, 1 mouse developed sclerosis to some extent. Fig. 6 shows damaged volume proportions (%) of both groups. On average, the volume proportion more significantly decreased in the case of the hydrogen water ingestion group than in the case of the control group. The above results revealed that drinking of hydrogen water results in arteriosclerosis prevention.

Example 3: Administration of a hydrogen gas to hyperlipidemia animal models and weight changes

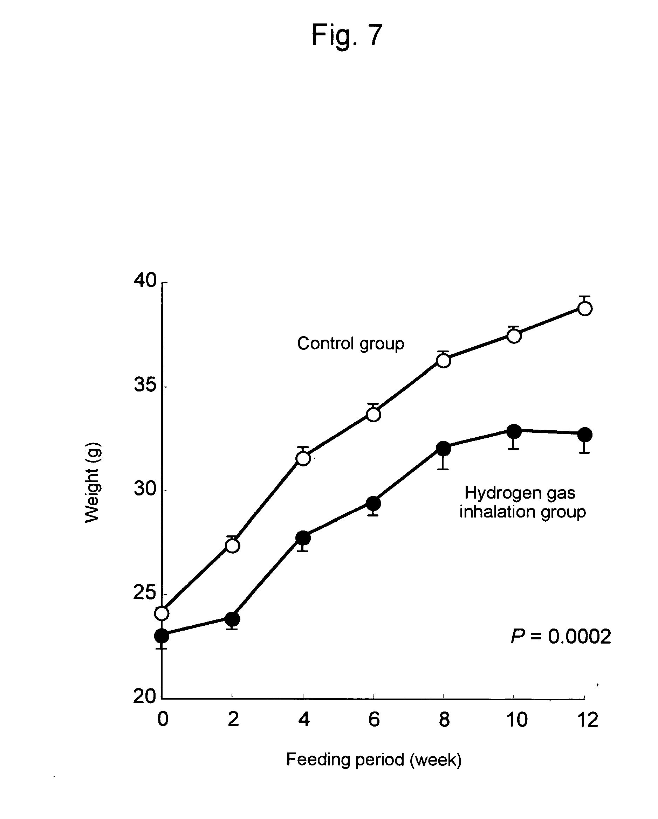

A hydrogen gas was administered to apoE knockout mice (apoE-KO mice), which are widely used as hyperlipidemia animal models. Then, effects on body fat and the liver were examined. 9-week-old male apoE-KO mice were placed in cages (4 mice each). One group was designated as a control group and normal air was supplied thereto. Another group was designated as a hydrogen gas inhalation group and a gas mixture containing a hydrogen gas (approximately 2%) was supplied thereto by the method described in "Administration of a hydrogen gas to mice" above. The control group was also housed in a similar plastic container. Fig. 7 shows weight changes following the administration of a hydrogen gas. In the hydrogen gas inhalation group, weight increases were significantly suppressed compared with those in the control group.

Measurement of muscle force

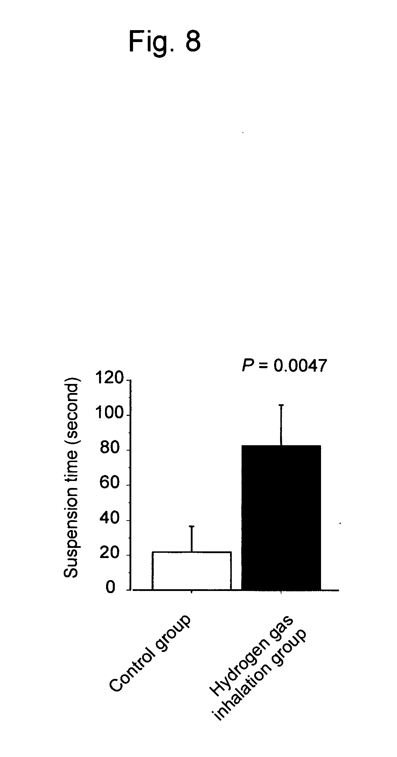

11 weeks after hydrogen administration, the mice were subjected to measurement of muscle force. Upon measurement, each mouse was allowed to hold a mesh wire of the ceiling and the time period until it fell from the ceiling was determined. Fig. 8 shows the results. Mice in the hydrogen gas inhalation group were able to suspend themselves for a significantly long time. This indicates that suppression of weight increases promotes maintenance of a healthy state. The fact that those in the control group easily fell down indicates that they had excessive weights.

CT analysis of body fat

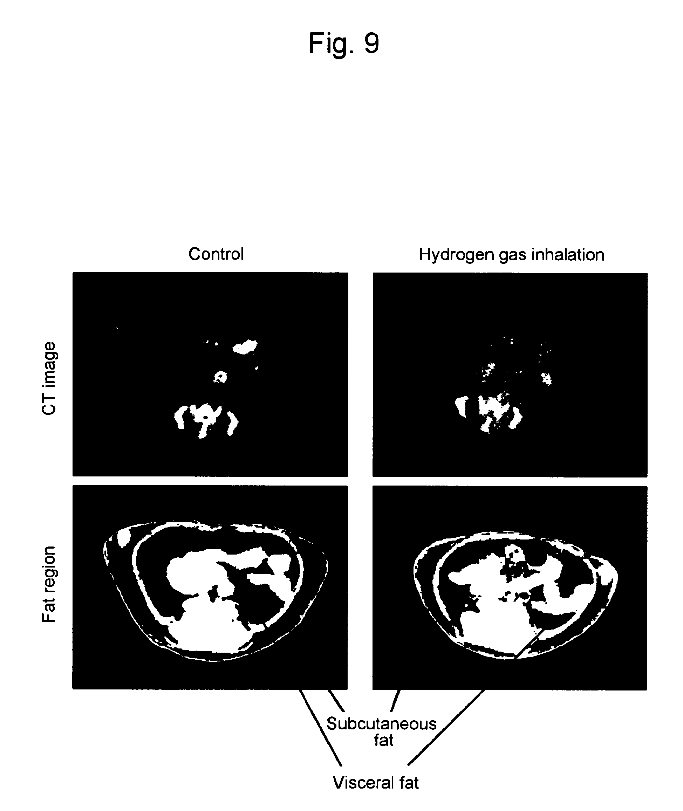

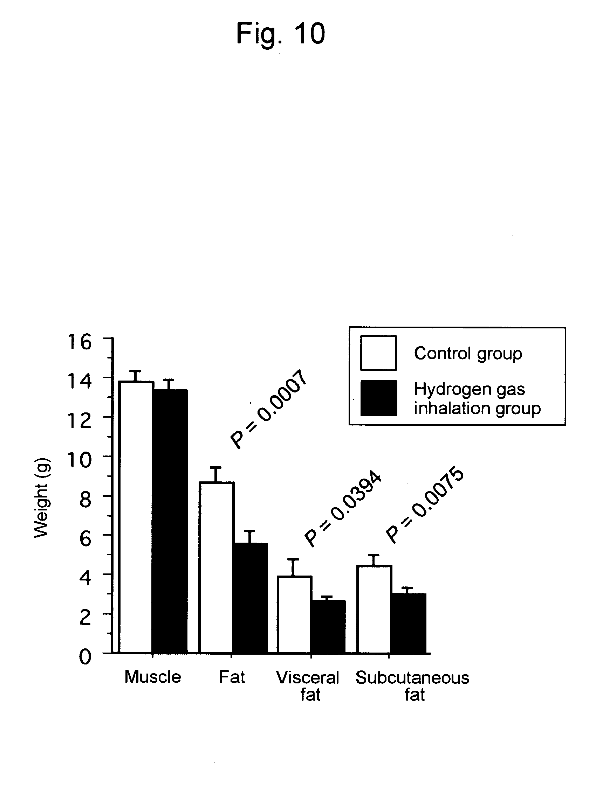

Next, each mouse was analyzed with an X-ray CT for laboratory animals (LaTheta LCT-100A, Aloka Co., Ltd.). General anesthesia induction was carried out by supplying an anesthetic gas mixture (oxygen: 0.3 L/min; nitrous-oxide gas: 0.7 L/min; Sevofrane: 3% (Maruishi Pharmaceutical Co., Ltd.)) to each mouse with the use of a general anesthesia apparatus for small animals (Soft Lander, NeuroScience, Inc). CT scanning was carried out under anesthesia so as to obtain whole-body images (fig. 9, upper images). Then, fat regions were determined based on CT values so as to specify subcutaneous fat and visceral fat (fig. 9, lower images). Each cross-sectional area was multiplied by the thickness so as to obtain the volume, followed by estimation of the fat and muscle weights. As a result, all the amounts of subcutaneous fat, visceral fat, and total fat were found to have significantly decreased in the hydrogen gas inhalation group (figs. 9 and 10). There was no difference in terms of muscle amount between the control group and the hydrogen gas inhalation group. It was shown that the fat only decreased in the hydrogen gas inhalation group. In addition, fat percentages were obtained by the following equation.

$$\text{Fat percentage (\%)} = \text{fat [visceral fat + subcutaneous fat]} / (\text{fat [visceral fat + subcutaneous fat]} + \text{muscle}) \times 100$$

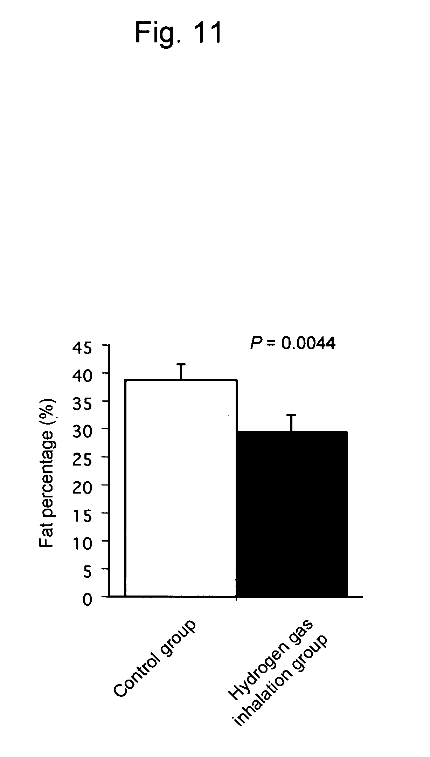

Fig. 11 shows the results. Also in this case, the fat percentage of the hydrogen gas inhalation group more significantly decreased than that of the control group, indicating decreases in body lipids.

Changes in the amount of fat accumulated in the liver

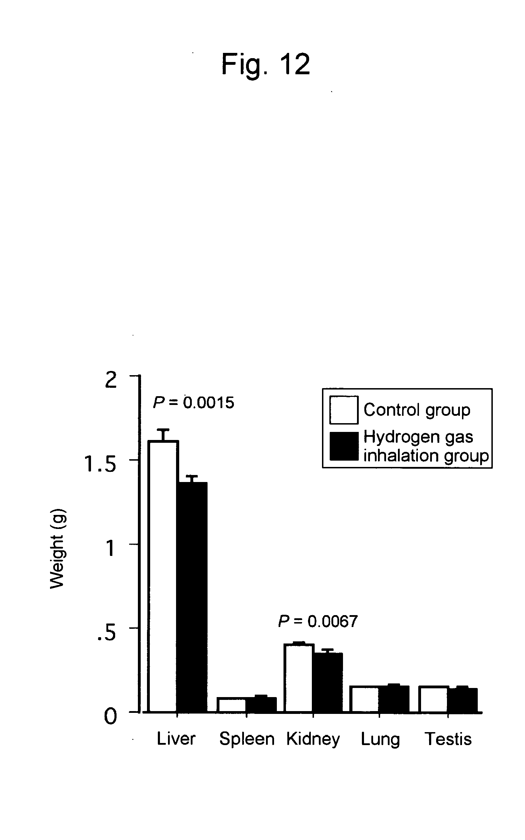

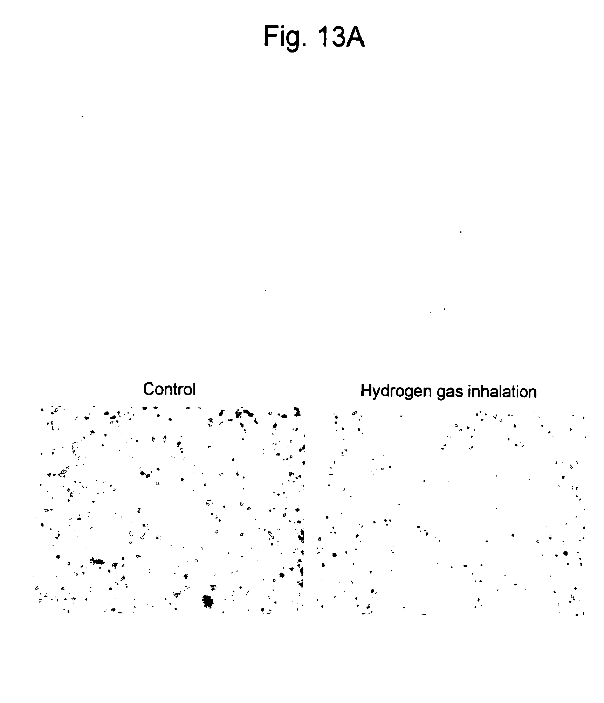

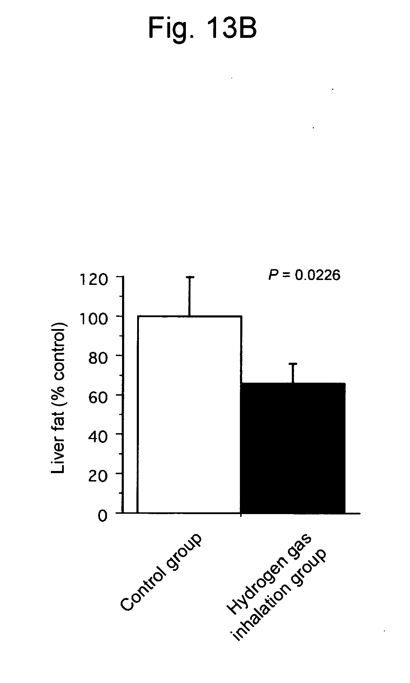

12 weeks after hydrogen gas inhalation, the mice were subjected to ether anesthesia, followed by exsanguination. The amounts of the following items in serum were measured: total cholesterol, LDL cholesterol, HDL cholesterol, neutral fat, AST, ALT, creatinine kinase, total protein, lactate dehydrogenase, urea nitrogen, total bilirubin, creatinine, alkaline phosphatase, choline esterase, and albumine. However, no difference was observed between the control group and the hydrogen gas inhalation group. As a result of weighing of the individual organs, the liver and kidney weights were found to have significantly decreased in the hydrogen gas inhalation group, as shown in fig. 12. Each liver was fixed with 4% paraformaldehyde overnight, followed by dehydration with sucrose. Thereafter, frozen sections were prepared and stained with Oil-Red O. Then, the amount of lipids accumulated in the liver was calculated (fig. 13A, images). The stained portions indicate fat accumulation. The graph in fig. 13B shows results of comparison of both groups, which were obtained by measuring stained areas with the use of free image processing software "NIH Image" based on digital images of the section surfaces. It was shown that the amount of fat accumulated in the liver more significantly decreased in the case of the hydrogen water ingestion group than in the case of the control group.
The above results clearly indicate that, as a result of inhalation of a hydrogen gas, abnormal fat accumulation found in apoE-KO mice is suppressed in each type of fat, such as subcutaneous fat, visceral fat, or fat in an organ, leading to improvement of physical strength as shown in muscle force measurement results.

Example 4: Determination of blood lipid metabolism improving effects obtained by hydrogen water ingestion

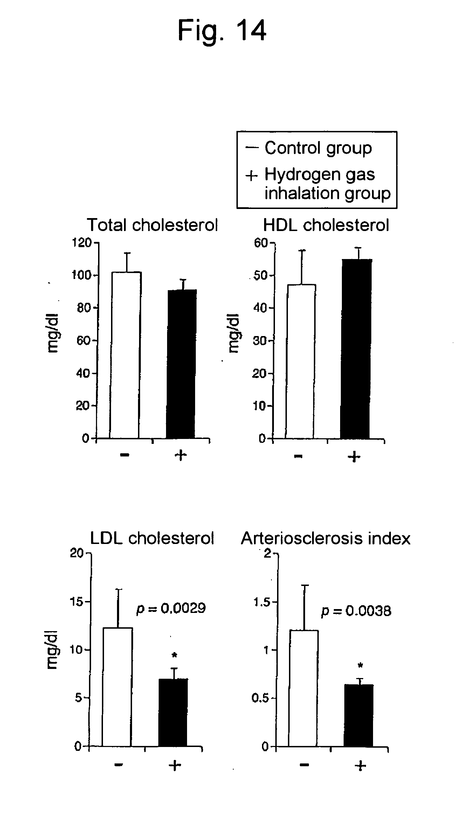

Normal mice (C57BL/6, male, 13 weeks old) were divided into groups of 8 mice. Among them, one group was fed ad libitum with hydrogen-free water (a control group), and another group was fed ad libitum with hydrogen water (a hydrogen water ingestion group). The method for administrating hydrogen water was the method described in "Administration of hydrogen water" above. 2 weeks later, weights, blood sugar levels, and blood cholesterol concentrations (total cholesterol (T-CHO), high density lipoprotein cholesterol (HDL-C), and low density lipoprotein cholesterol (LDL-C)) were measured. There was no difference between both groups in terms of weights and blood sugar levels. However, LDL cholesterol and the arteriosclerosis index more significantly decreased in the case of the hydrogen water ingestion group than in the case of the control group. Herein, the arteriosclerosis index was obtained by the following equation. Fig. 14 shows the results.

$$\text{Arteriosclerosis index} = [(\text{total cholesterol}) - (\text{HDL-C})] \div (\text{HDL-C})$$

The results shown in fig. 14 indicate that hydrogen water ingestion caused a decrease in LDL cholesterol (bad cholesterol) capable of increasing excessive cholesterol in blood vessels and an increase in the proportion of HDL cholesterol (good cholesterol) capable of removing cholesterol in blood vessels with respect to total cholesterol. Also, the results indicate that hydrogen water is effective for prevention of hyperlipidemia and of arteriosclerosis and heart diseases caused by hyperlipidemia serving as a risk factor.

Industrial Applicability

The composition containing hydrogen molecules of the present invention has the above constituent features, and thus it suppresses body fat accumulation and arteriosclerosis, as described in details in the Examples. Further, it decreases low density lipoprotein (LDL) cholesterol in blood. That is, it can improve lipid metabolism. The composition containing hydrogen molecules of the present invention exhibits its effects in a liquid or gas state, and thus it can be used as a pharmaceutical composition for lipid metabolism improvement and for a beverage such as a health beverage.

All publications, patents, and patent applications cited herein are incorporated herein by reference in their entirety.

**Claims**

1.  A lipid metabolism improving agent comprising a liquid containing at least hydrogen molecules.

2.  The lipid metabolism improving agent according to claim 1, wherein the liquid containing at least hydrogen molecules is an aqueous solution.

3.  The lipid metabolism improving agent according to claim 2, which contains hydrogen molecules at 0.1 ppm (0.05 mM) or more.

4.  The lipid metabolism improving agent according to claim 2 or 3, which further contains oxygen molecules.

5.  A lipid metabolism improving agent comprising a gas containing at least hydrogen molecules.

6.  The lipid metabolism improving agent according to claim 5, wherein the gas containing at least hydrogen molecules is a gas mixture of a hydrogen gas and an oxygen gas.

7.  The lipid metabolism improving agent according to claim 5, wherein the gas containing at least hydrogen molecules is a gas mixture of a hydrogen gas, an oxygen gas, and an inert gas.

8.  The lipid metabolism improving agent according to claim 5, wherein the gas containing at least hydrogen molecules is a gas mixture of a hydrogen gas and air.

9.  The lipid metabolism improving agent according to any one of claims 5 to 8, which contains a hydrogen gas at a concentration of 1% to 4% (v/v).

10. The lipid metabolism improving agent according to any one of claims 5 to 8, which contains a hydrogen gas at a concentration of 1.0% to 4.5% (v/v).

11. The lipid metabolism improving agent according to any one of claims 1 to 10, which suppresses body fat accumulation.

12. The lipid metabolism improving agent according to any one of claims 1 to 10, which suppresses arteriosclerosis.

13. The lipid metabolism improving agent according to any one of claims 1 to 10, which reduces low density lipoprotein (LDL) cholesterol in blood.

14. A beverage suitable for lipid metabolism improvement, which comprises a liquid containing at least hydrogen molecules.

15. The beverage suitable for lipid metabolism improvement according to claim 14, wherein the liquid containing at least hydrogen molecules is an aqueous solution.

16. The beverage suitable for lipid metabolism improvement according to claim 15, which contains hydrogen molecules in an oversaturated state.

17. The beverage suitable for lipid metabolism improvement according to claim 14 or 15, which further contains oxygen molecules.

**18.** The beverage according to any one of claims 14 to 17, which is labeled for use for improving lipid metabolism.

**19.** The beverage according to claim 18, which is labeled for use for suppressing body fat accumulation.

**20.** The beverage according to claim 18, which is labeled for use for suppressing arteriosclerosis.

**21.** The beverage according to claim 18, which is labeled for use for reducing low density lipoprotein (LDL) cholesterol in blood.

**22.** The beverage according to any one of claims 14 to 21, which corresponds to a health food, a functional food, a nutritional supplement food, a supplement, or a specified health food.

**23.** A container containing the lipid metabolism improving agent according to any one of claims 5 to 10.

**24.** The container according to claim 23, which is a hydrogen gas cylinder.

**25.** An apparatus for supplying the lipid metabolism improving agent comprising a gas containing at least hydrogen molecules according to any one of claims 5 to 10 to a test subject that needs to improve lipid metabolism, which comprises a container containing the lipid metabolism improving agent, a gas inhalation means, and a supply pipe for supplying a gas in the container to the inhalation means.

**26.** The apparatus for supplying the lipid metabolism improving agent to a test subject that needs to improve lipid metabolism according to claim 25, which comprises a container containing at least one type of gas selected from the group consisting of an oxygen gas, an inert gas, and air, provided that the lipid metabolism improving agent comprising a gas containing at least hydrogen molecules and at least one type of gas selected from the group consisting of an oxygen gas, an inert gas, and air are supplied separately or in a mixed state to the gas inhalation means.

**27.** The apparatus for supplying the lipid metabolism improving agent to a test subject that needs to improve lipid metabolism according to claim 25 or 26, which contains a hydrogen gas at 1% to 4% in the container containing the lipid metabolism improving agent comprising a gas containing at least hydrogen molecules.

**28.** The apparatus for supplying the lipid metabolism improving agent to a test subject that needs to improve lipid metabolism according to any one of claims 25 to 27, wherein the container containing the lipid metabolism improving agent comprising a gas containing at least hydrogen molecules is a gas cylinder.

**29.** The apparatus for supplying the lipid metabolism improving agent comprising a gas containing at least hydrogen molecules to a test subject that needs to improve lipid metabolism according to any one of claims 25 to 28, wherein the gas inhalation means is a gas inhalation mask.

**30.** The apparatus for supplying the lipid metabolism improving agent to a test subject that needs lipid metabolism improvement according to any one of claims 25 to 28, wherein the gas inhalation means is a sealed chamber, provided that the lipid metabolism improving agent comprising a gas containing at least hydrogen molecules is supplied to the sealed chamber such that the lipid metabolism improving agent is supplied to the test subject in the sealed chamber.

# Fig. 1

# Fig. 2

# Fig. 3

High-fat diet      Ordinary diet

Hydrogen water      Control

# Fig. 4

# Fig. 5

Control        Hydrogen water ingestion

# Fig. 6

# Fig. 7

# Fig. 8

# Fig. 9

Control      Hydrogen gas inhalation

CT image

Fat region

Subcutaneous fat

Visceral fat

# Fig. 10

# Fig. 11

# Fig. 12

# Fig. 13A

Control                    Hydrogen gas inhalation

# Fig. 13B

$P = 0.0226$

Liver fat (% control)

Control group

Hydrogen gas inhalation group

# Fig. 14

| — Control group |
| + Hydrogen gas inhalation group |

Total cholesterol

HDL cholesterol

LDL cholesterol

p = 0.0029

Arteriosclerosis index

p = 0.0038

# Fig. 15

<table>
<tr><td colspan="2" align="center">**INTERNATIONAL SEARCH REPORT**</td><td>International application No.<br>PCT/JP2007/067458</td></tr>
</table>

**A. CLASSIFICATION OF SUBJECT MATTER**
*A61K33/00*(2006.01)i, *A23L1/30*(2006.01)i, *A23L2/38*(2006.01)i, *A61K9/08* (2006.01)i, *A61K9/72*(2006.01)i, *A61P3/06*(2006.01)i, *A61P9/10*(2006.01)i

According to International Patent Classification (IPC) or to both national classification and IPC

**B. FIELDS SEARCHED**

Minimum documentation searched (classification system followed by classification symbols)
A61K33/00, A23L1/30, A23L2/38, A61K9/08, A61K9/72, A61P3/06, A61P9/10

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), JMEDPlus(JDream2), JST7580(JDream2), JSTPlus(JDream2)

**C. DOCUMENTS CONSIDERED TO BE RELEVANT**

| Category* | Citation of document, with indication, where appropriate, of the relevant passages | Relevant to claim No. |
|---|---|---|
| P,X | WO 2007/021034 A1 (Ikuro OSAWA),<br>22 February, 2007 (22.02.07),<br>Full text<br>(Family: none) | 1-30 |
| P,X | WO 2006/120747 A1 (Wataru MUROTA),<br>16 November, 2006 (16.11.06),<br>Full text<br>& WO 2006/120761 A1 | 14-22 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>16 October, 2007 (16.10.07) | Date of mailing of the international search report<br>23 October, 2007 (23.10.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| | International application No. |
| --- | --- |
| | PCT/JP2007/067458 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>A | WO 2005/039602 A1  (Miz Co., Ltd.),<br>06 May, 2005 (06.05.05),<br>Full text; particularly, pages 10 to 13,<br>Par. Nos. [0043] to [0047]; pages 23 to 24,<br>Par. Nos. [0093] to [0098]; page 25, Par. No.<br>[0103]; examples; page 146, Par. No. [0556];<br>Claims<br>& JP 2005-126384 A | 1-4,11-22<br>5-10,23-30 |
| X<br>A | WO 2003/002466 A1  (Miz Co., Ltd.),<br>09 January, 2003 (09.01.03),<br>Full text; particularly, page 1, lines 22 to<br>25; page 10, line 27 to page 13, line 9;<br>page 24, lines 8 to 25; examples; Claims<br>& US 2004/154993 A1     & EP 1413555 A1<br>& WO 2004/039735 A1    & BR 210726 A<br>& CA 2452682 A            & CN 1522229 A | 1-4,11-22<br>5-10,23-30 |
| X<br>A | JP 2005-87257 A  (Sinwa Co., Inc.),<br>07 April, 2005 (07.04.05),<br>Full text; particularly, Claims; page 3,<br>Par. No. [0003]; page 4, Par. Nos. [0010],<br>[0012]; pages 5 to 6, examples<br>(Family: none) | 1-25,27-29<br>26,30 |
| X<br><br>A | JP 3-504566 A  (Essaidi, Mohammed),<br>09 October, 1991 (09.10.91),<br>Full text; particularly, Claims; page 3, lower<br>left column, line 11; page 3 lower right<br>column, line 11 to page 4, lower right column<br>& EP 417201 A1           & WO 1989/011267 A1<br>& NL 8801360 A           & BR 8907453 A<br>& AU 639415 B            & OA 9331 A | 5-13,23,<br>25-27,30<br>1-4,14-22,<br>24,28,29 |
| X<br>A | JP 2005-13833 A  (Ecojapan Co., Ltd.),<br>20 January, 2005 (20.01.05),<br>Full text; particularly, Claims; page 8,<br>Par. No. [0046]<br>(Family: none) | 14-22<br>1-13,23-30 |
| X<br>A | JP 2002-301483 A  (Shigemi IIDA),<br>15 October, 2002 (15.10.02),<br>Full text; particularly, Claims<br>(Family: none) | 14-22<br>1-13,23-30 |
| X<br>A | JP 2005-21146 A  (Hiroshima Kasei Kabushiki<br>Kaisha),<br>27 January, 2005 (27.01.05),<br>Full text; particularly, Claims; examples<br>(Family: none) | 14-22<br>1-13,23-30 |

Form PCT/ISA/210 (continuation of second sheet) (April 2005)

**INTERNATIONAL SEARCH REPORT**

International application No.

PCT/JP2007/067458

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | JP 2005-296794 A  (T. E. D Inc., Ltd.),<br>27 October, 2005 (27.10.05),<br>Full text; particularly, Claims<br>& JP 3-606466 B        & US 2005/224996 A1 | 14-22<br>1-13,23-30 |
| X<br>A | JP 2005-87191 A  (Wataru MUROTA),<br>07 April, 2005 (07.04.05),<br>Full text; particularly, Claims; page 3,<br>Par. No. [0011]<br>(Family: none) | 14-22<br>1-13,23-30 |
| X<br>A | JP 2004-329186 A  (Wataru MUROTA),<br>25 November, 2004 (25.11.04),<br>Full text; particularly, Claims; page 3,<br>Par. No. [0010]<br>(Family: none) | 14-22<br>1-13,23-30 |
| X<br>A | JP 2003-170178 A  (Hitachi Zosen Tomioka Kikai<br>Kabushiki Kaisha),<br>17 June, 2003 (17.06.03),<br>Full text; particularly, Claims; page 3,<br>Par. No. [0016]<br>(Family: none) | 14-22<br>1-13,23-30 |
| X<br>A | JP 2003-19426 A  (Yugen Kaisha Joho Kagaku<br>Kenkyusho),<br>21 January, 2003 (21.01.03),<br>Full text; particularly, Claims; examples<br>(Family: none) | 14-22<br>1-13,23-30 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002322052 A
- JP 2002326932 A
- JP 2005087257 A
- JP 2000517311 A

- JP 20052385725 PCT
- JP 2006157827 PCT
- JP 2006235922 A

**Non-patent literature cited in the description**

- *FASEB J.,* 2001, vol. 15, 2623-2630

- *Circulation,* 2004, vol. 110, 1492-1498